(19) Europäisches Patentamt

European Patent Office

Office européen des brevets

(11) **EP 0 820 516 B1**

(12) **EUROPEAN PATENT SPECIFICATION**

(45) Date of publication and mention
of the grant of the patent:
**06.03.2002 Bulletin 2002/10**

(51) Int Cl.⁷: **C12N 15/56**, C12P 21/00,
C12N 9/24, C02F 3/00,
B01D 61/00, C12N 1/20
// (C12N1/20, C12R1:465)

(21) Application number: **96912008.8**

(22) Date of filing: **09.04.1996**

(86) International application number:
**PCT/EP96/01530**

(87) International publication number:
**WO 96/31610 (10.10.1996 Gazette 1996/45)**

(54) **EXOPOLYSACCHARIDE DEGRADING ENZYME AND USE OF THE SAME**

EXOPOLYSACCHARID ABBAUENDES ENZYM UND VERWENDUNG DAVON

ENZYME CAPABLE DE DEGRADER DES EXOPOLYSACCHARIDES ET SON UTILISATION

(84) Designated Contracting States:
**AT BE CH DE DK ES FI FR GB IE IT LI LU NL SE**

(30) Priority: **07.04.1995 EP 95105311**
**14.09.1995 US 527905**

(43) Date of publication of application:
**28.01.1998 Bulletin 1998/05**

(73) Proprietor: **BetzDearborn, Inc.**
**Trevose, PA 19053 (US)**

(72) Inventors:
• **VAN SPEYBROECK, Michel, M., P.**
**B-9000 Gent (BE)**
• **BRUGGEMAN, Geert**
**B-9910 Knesselare (BE)**
• **VAN POELE, Jozef**
**B-2140 Borgerhout (BE)**
• **VAN PEE, Kristine, Laura, Ignatius**
**B-9880 Aalter (BE)**
• **VANDAMME, Erick, J.**
**B-9032 Wondelgem (BE)**

(74) Representative: **UEXKÜLL & STOLBERG**
**Patentanwälte Beselerstrasse 4**
**22607 Hamburg (DE)**

(56) References cited:
• **ZENTRALBL BAKTERIOL [ORIG A], FEB 1974, 226 (1) P26-35, GERMANY, WEST, XP000579261 STIRM S ET AL: "[On a bacteriophage-induced colanic acid depolymerase (author's transl)]"**
• **CURR MICROBIOL, 26 (5). 1993. 299-304., XP000579185 OSMAN S F ET AL: "ISOLATION AND CHARACTERIZATION OF AN EXOPOLYSACCHARIDE DEPOLYMERASE FROM PSEUDOMONAS-MARGINALIS HT041B"**
• **CURR MICROBIOL, 18 (5). 1989. 323-330., XP000579186 NGUYEN L K ET AL: "IDENTIFICATION OF A SLIME EXOPOLYSACCHARIDE DEPOLYMERASE IN MUCOID STRAINS OF PSEUDOMONAS-AERUGINOSA"**
• **EUR J BIOCHEM, DEC 10 1971, 23 (3) P582-7, GERMANY, WEST, XP000579315 SUTHERLAND IW: "Enzymic hydrolysis of colanic acid."**

**Description**

The present invention relates to an exopolysaccharide degrading enzyme prepared from a newly isolated strain of *Streptomyces* GDR-7 (ATCC 55601) having a molecular weight of about 100 kDa and being capable of degrading colanic acid, to enzymatic compositions capable of degrading exopolysaccharides and in particular to the reduction or removal of biofilm on surfaces and to the prevention of biofilm formation on such surfaces. More particularly, the present invention relates to the use of said enzyme and/or the enzymatic composition comprising this enzyme, for the reduction, removal and/or prevention of biofilm on surfaces of aqueous systems (water-bearing systems, fluid systems). The present invention further relates to the Streptomyces strain GDR-7 (ATCC 55601) producing the exopolysaccharide degrading enzyme.

Attachment of micro-organisms to solid surfaces is common in aqueous systems. Generally this phenomenon is termed biofouling; the layer of microorganisms is termed biofilm. Biofilm accumulation is the result of processes involving: 1) transport of material from the bulk fluid to the surface and subsequent attachment, 2) microbial metabolism within the biofilm, 3) fluid shear stress at the film surface, 4) surface material and roughness, 5) fouling control procedures.

Problems associated with biofilm formation in different industrial processes are energy losses, material deterioration and reduced process effectiveness. Energy losses mean reduced heat exchanging capacity in cooling towers and increased power consumption in fluid distribution systems and in shipping industry. Material deterioration, caused by the biofilm layer next to the solid surface, means corrosion and rot. Reduced process effectiveness is seen in water treatment, pulp and paper industry and water quality data collection.

Health care also is involved with biofouling, e.g. formation of dental plaque, attachment of microbial cells to eukaryotic tissues causing disease, quality of drinking water, release of pathogenic organisms from biofilms into industrial water systems. Industrial process- or operating-water systems, such as e.g. open or closed water-cycle systems of paper factories or cooling-water systems, offer suitable conditions for the growth of microorganisms, with the result that biofilm (or slime) is formed on surfaces of water-bearing systems. In the case of cooling-water systems in particular, these biofilm deposits can lead to a reduced heat exchange, damage to the joints of pipelines and corrosion within the systems. In this way adverse effects on process control are possible, which can reduce the efficiency of the industrial process in question or impair product quality. In addition to this, biofilm or slime deposits generally lead to higher energy consumption. Most affected by increased biofilm formation are industrial processes such as the manufacture of pulp, paper, board and textiles. In the case of paper machines for example, fairly large quantities of water are recirculated in cycle systems called "white water systems" (primary or secondary cycle, i.e. white water I or II). The white water which contains dispersed pulp forms an ideal culture medium for the growth of microorganisms.

Apart from industrial aqueous systems, biofilm formation also occurs on surfaces in other environments, such as on ultrafiltration and dialysis membranes in health care. Within the scope of the invention the enzymatic composition can be utilized for slime prevention and removal in any system in which biofilm formation occurs, wherein the use for the treatment of the human or animal body by therapy practised on the human or animal body is excluded.

Biofilm or slime are formed by bacteria, in particular gram-negative bacteria, such as *Pseudomonas, Acinetobacter* and *Aerobacter* plus *Flavobacterium, Desulfovibrio, Escherichia, Sphaerotilus, Enterobacter* and *Sarcina.* The cell-wall structure of gram-negative bacteria is a factor which contributes particularly to slime formation. The cell wall comprises peptidoglycan, which consists of acetyl amino sugars and amino acids plus an outer membrane composed of proteins, lipopolysaccharides and lipoproteins. In contrast, the cell wall of gram-positive bacteria, e.g. *Bacillus,* is mostly composed of peptidoglycan and teichonic acids.

Biofilm is further produced by fungi and yeasts, such as *Pullularia pullulans, Alternaria sp, Lenzytes, Lentinus, Polyporus, Fomes, Sterium, Aspergillus, Fusarium, Penicillium, Candida, Saccharomyces* and *Basidomycetes.*

A biofilm can comprise a variety of micro-organisms. Within a biofilm, species of gram-negative and gram-positive bacteria, fungi, and algae may be found. Development of a biofilm is initiated by the concentration of organic molecules, i.e. lipids, proteins, sugars on an inert surface. Attraction of micro-organisms to this layer and subsequent adhesion through exopolymers then occurs. The attached micro-organisms then form discrete microcolonies. When after a while more colonies grow into each other, a true biofilm is formed. The biofilm becomes thicker until a steady state is reached: attraction of micro-organisms from fluid to the existing biofilm is compensated by the shearing of micro-organisms from biofilm to flowing fluid.

The thickness of a biofilm increases with substrate concentration. Within a thick biofilm certain regions may be depleted of nutrients resulting in weak structures. These weak spots can detach creating holes in the biological matrix. Subsequent action of flow on these holes can detach more material leaving a thin biofilm. As the biofilm becomes thicker, an anaerobic area near to the surface develops. In this area microbes are able to destruct the surface.

Generally, micro-organisms in biofilm are surrounded by copious amounts of extracellular biopolymers termed glycocalyx. The glycocalyx is defined as "any polysaccharide, containing bacterial surface structure that is distal to the surface of the outer membrane of gram-negative bacteria, or to the surface of the peptidoglycan layer of gram-positive bacteria". These extracellular polysaccharides are known as exopolysaccharides (EPS).

The glycocalyx can consist of regularly arranged glycoproteins, termed S-layer, at the cell wall, or of a fibrous polysaccharide matrix, capsule, at the cell surface that may partially be shed into the menstruum. This capsule can be highly organized. Sometimes it is seen that the polysaccharide capsule surrounding the microbe is not covalently attached to the cell surface. Pelleting the cells then leaves the glycocalyx in the supernatant.

Glycocalyx-enclosed microcolonies are formed by cell replication occurring so that both daughter cells are trapped within the same glycocalyx. Intermolecular binding of glycocalyx biopolymers is affected by divalent cations. Chelation of these cations with EDTA is effective in detaching biofilm.

Several workers have drawn a general conclusion concerning the function of the bacterial glycocalyx as known today. It has a function

1) in adhesion of cells to solid surfaces or to other, prokaryotic or eukaryotic, cells and
2) in trapping organic nutrients from the medium.
3) A capsule can be sufficiently highly organized to exclude particles and so protect the micro-organism from the environment. One can think of the glycocalyx as a first defensive wall against antibiotics, antibodies, bacteriophages.

Biofilms seldom consist of microbial material alone. Often inorganics are part of the slime, e.g. $CaCO_3$, alumina, silica, iron, magnesium, copper. In paper mills a lot of material can be included in the film, e.g. fibres, fillers, pitch, rosin size etc.

The deposition of bacterial slimes can most effectively be controlled with biocides, the effect of these biocides being based on the fact that they kill the microorganisms in the operating water and thus prevent slime production. However, biofilm producing bacteria are far more resistant to toxicants than planktonic bacteria. Therefore, very high concentrations of biocides are necessary to remove biofilm. This is because biofilm cells are slow-growing and metabolically less active and because they are protected by their glycocalyx, which not only acts as an ion-exchange resin immobilizing toxicants, but also as a hydrophobic/hydrophilic barrier, preventing biocides from reaching the cell. Further, biocides raise concerns on ecological grounds and, because of their toxicity, create considerable problems when handled. For this reason, alternative ways of eliminating biofilm were sought in the past, with particular attention being paid to enzymes.

The biofilm matrix can be heterogeneous; it is primarily built up from polysaccharides. Research in the field of slime removal has thus concentrated in particular on studies of enzymes that hydrolyze polysaccharides (hereinafter referred to as polysaccharidases). The use of enzymes, such as polysaccharidases, to degrade the glycocalyx and thus to remove biofilm or to prevent slime formation in industrial water systems is well known in the art.

Several approaches have already been suggested for this purpose, based on the different views with respect to the composition of industrial slime or biofilm, respectively.

A first approach is the use of a lytic enzyme, not active against the excreted exopolysaccharides in the slime, but against polysaccharides in the cell walls. These enzymes thus destroy cell walls and kill bacteria. For example, in DE 37 41 583, the use of a mixture consisting of glucanase and protease having lytic enzyme activity against 1,3-glucose linkages in the cell walls is disclosed. However, the slime layer protecting the bacterial cells can prevent the enzymes from reaching the cell walls.

A second approach considers industrial slime as being composed of a single polysaccharide type, produced by one bacterial species. For example, in US 3,824,184 and US 3,773,623 the use of a levan hydrolase, which breaks down levan, produced by a wide variety of bacteria, is disclosed. Levan is, however, only produced by bacteria growing on sucrose. With regard to paper mills or cooling systems, it is unlikely that sucrose is present in significant amounts, so that levan will not be an important component of biofilms.

CA 1,274,442 and WO 90/02794 disclose the use of the enzyme alginate lyase, degrading alginate which is produced mainly by *Pseudomonas* spp. However, *Pseudomonas* spp. mostly do not produce big amounts of alginate lyase under field conditions and the use of an alginate lyase as such is of limited value.

Further, industrial slime is always produced by a population consisting of different microorganisms, which can vary depending on the industrial site. Industrial slime (biofilm) will never be composed of one single exopolysaccharide since each microorganism produces its own typical exopolysaccharide (EPS).

In US 4,055,467 the use of a pentosanase-hexosanase for preventing biofilm formation in a cooling tower has been disclosed.

A third approach starts from the fact that a lot of different heteropolysaccharides are present in industrial slime. It is well known in the art that these polysaccharides are mainly composed of glucose, galactose, mannose, fucose, rhamnose, ribose, glucosamine, galactosamine, mannuronic acid, galacturonic acid and glucuronic acid in a very complex arrangement (cf. L. Kenne et al. in G. Aspinall (ed.) "The Polysaccharides", vol. II (1983), Academic Press; I.W. Sutherland in "Surface Carbohydrates of the Procaryotic Cell", Academic Press, London, 1977, 27-96).

Further, numerous other sugar components are present in smaller quantities.

Therefore, it could be assumed that a lot of different enzyme activities are to be combined to have some effect on industrial slime. The knowledge of the monosaccharide composition of slime is, however, not sufficient for the definition of an enzyme mixture which is successful to remove biofilm. The respective monosaccharides can be linked in many different ways. Glucose for instance can be alpha-1,2, alpha-1,3, alpha-1,4, alpha-1,6, beta-1,2, beta-1,3, beta-1,4 or beta-1,6 linked. For each of these, a separate enzymatic activity could be added. Also, adjacent monosaccharides and the substitutents on the saccharides are very important for the activity of certain polysaccharidases.

Further, one would normally expect that a single polysaccharidase, with one of the many possible activities against one of the main saccharide building blocks of a polysaccharide occuring in biofilm, or even a mixture of a few polysaccharidases, would have no or only a very limited effect on the complex composition of heteropolysaccharides in biofilm. However, it has been found in the art that some complex mixtures develop a positive effect on the degradation of heteropolysaccharides. US 5,071,765 and EP-A-0 388 115 relate to the use of mixtures of cellulase, alpha-amylase and a protease, respectively, attacking beta- and alpha-1,4-linked glucose and extracellular protein. It is disclosed that the amylase nicks the outside of the slime molecules, thereby allowing the cellulase to enter the structure and degrade the slime. It was, however, never proven that slime would have alpha-1,4-linkages specifically at the outside and beta-1,4-linkages at the inside.

In US 5,238,572 a combination of enzymes selected from the group consisting of galactosidase, galacturonidase, rhamnosidase, xylosidase, fucosidase, arabinosidase and alpha-glucosidase is disclosed.

A fourth approach to prevent biofilm formation is to control the initial step of slime formation, i.e. the adhesion of bacteria. In EP-A-0 425 017 it is disclosed that microorganisms are bound to a surface, in part, by linkages reactive with Type II endoglycosidases. These types of enzymes (endo-beta-N-acetylglucosaminidases, endo-alpha-N-acetylgalactosaminidases and endo-beta-N-acetylgalactosidases) are capable of cleaving specific internal glycosidic linkages found in glycoproteins. It is known that some of these enzymes are also lytic.

Many different enzymatic methods have been proposed in the art for the removal of biofilm or biofilm prevention which either required a combination of numerous enzymes or, as far as only one or few enzymes were used, these had only a limited range of action. In addition, these approaches failed to provide a composition which, apart from removing or controlling slime, also prevented bacterial adhesion to surfaces of aqueous systems and effected detachment of adhered bacteria.

The problem underlying the present invention is therefore to provide an enzyme composition or enzyme with a broad range of action. Preferably the composition should also be capable of both preventing attachment of bacteria and detaching bacteria which are already adhered to the surfaces of the system. In particular, an agent should be provided which contains only one enzyme or a simple mixture of very few enzymes, respectively. Advantageously, the composition should not contain any biocide. The. enzyme or enzymes should have an activity which allows the application of smaller amounts of enzymes than known for enzymes or enzyme mixtures in the art.

The object of the present invention is therefore to make available a composition and a process for the prevention of slime formation and for the reduction and/or removal of biofilm on surfaces of aqueous systems which avoid the disadvantages of conventional biocides but achieves or exceeds their degree of effectiveness, respectively.

According to the invention, the problem is solved by providing a new exopolysaccharide degrading enzyme from the *Streptomyces* strain GDR-7 (ATCC 55601) having a molecular weight of about 100 kDa and being capable of degrading colanic acid, and an enzymatic composition comprising this enzyme, said enzyme and composition being capable of degrading exopolysaccharides produced by bacteria found in water systems, especially bacteria from the *Enterobackeriaceae* group. The enzyme and enzymatic compositions of the present invention are useful in preventing slime formation and/or reducing and/or removing biofilm on surfaces of aqueous systems.

As used herein, the reduction, removal and/or prevention of biofilm includes the reduction and/or removal of exopolysaccharides in biofilm and/or the prevention of the attachment of exopolysaccharides on surfaces of aqueous systems.

*Enterobacteriaceae* are ubiquitous microorganisms that can build up large amounts of slime either as a capsule or as loose slime, associated with these bacteria. *Enterobacteriaceae* are occurring in paper mills as well as in cooling water (Drew Chemical Corporation, "Principles of Industrial Water Treatment", 1978, 2nd ed., p. 90) or in drinking water distribution systems (Lechevallier et al., Appl. & Env. Microb. 53, 1987, 2714-2724). Bacteria of this group are known to produce exopolysaccharides, including the M-antigen polysaccharide known as colanic acid (Aspinall, "The Polysaccharides", Academic Press (1983), 314).

According to the invention it has surprisingly been found that a composition comprising the exopolysaccharide degrading enzyme from the *Streptomyces* strain GDR-7 (ATCC 55601) having a molecular weight of about 100 kDa, as determined by sodium dodecyl sulfate polyacrylamide gel electrophoresis (SDS-PAGE) under denaturing conditions, and being capable of degrading colanic acid has broad activity and is active against numerous microorganisms of different types.

Preferably, this enzyme is produced by cultivating the *Streptomyces* strain GDR-7 in a suitable medium. This strain has been deposited at the American Type Culture Collection (ATCC), 12301 Parklawn Drive, Rockville, MD 20852 USA, on July 27, 1994 under the Budapest Treaty, the deposit corresponding to designation ATCC 55601.

The enzyme obtained from the above-referenced bacteria can be used for the prevention of slime formation and/or reduction and/or removal of biofilm, either in the form of a crude enzyme culture supernatant or in its purified form (i.e., the enzyme is separate from the cells and the medium). Suitable methods for the recovery and purification of enzymes are well known in the art.

In a preferred embodiment of the invention, the enzyme is prepared by cultivating the *Streptomyces* strain GDR-7, corresponding to ATCC designation 55601, or a cell strain derived therefrom in a suitable medium. Mutants, variants or transformants of the *Streptomyces* strain ATCC 55601, which are capable of producing said enzyme, are also preferred according to the present invention.

It is further possible to clone and express the gene or fragments thereof encoding the exopolysaccharidase degrading enzyme from the *Streptomyces* strain ATCC 55601 in a suitable host cell, especially bacterial, plant or animal cells.

As used herein, the term "enzyme" includes biologically active analogs, (natural and synthetic) variants, fragments and chemically modified derivatives of the enzyme, which are capable of degrading exopolysaccharides. According to the present invention, the primary, secondary and/or tertiary structure of the enzyme can be modified as long as its biological activity is retained.

According to the present invention, the exopolysaccharidase (either purified or in crude form) is also active against other bacterial strains, such as *Klebsiella, Pseudomonas, Xanthomonas* etc. This activity therefore contributes to the effectiveness of the enzymatic composition in biofilm removal in systems which contain a variety of bacterial species besides *Enterobacteriaceae.*

Within the scope of the present invention, the enzymatic composition can further comprise at least one enzyme from the group consisting of polysaccharidases, proteases, lipases and glycoproteases. In one embodiment, the additional use of at least one protease, for example from the group consisting of serine protease produced by *Bacillus licheniformis* (this enzyme is often referred to as alkaline protease); protease type XXI of *Streptomyces griseus*, and metalloprotease of *Bacillus polymyxa* type IX, is preferred. Most widely employed proteases in cleaning compositions are the alkaline proteases derived from various strains of *Bacillus.* Such proteases, which are marketed under tradenames such as Esperase® and Alcalase® from NOVO Nordisk Bioindustrials Inc. and Maxatase® and Maxacal® from Gist-Brocades have desirable alkaline stability properties and proteolytic activities, useful for combining with EPS'ase. In particular, a combination of exopolysaccharidase with *Aspergillus* acid proteinase of *Aspergillus saitoi*, is most advantageous.

Further, the composition may also comprise at least one enzyme stabilizing agent, in particular an agent selected from the group consisting of glycerol, sorbitol, glucose and EDTA.

The enzymatic compositions of the invention are successfully used in processes for avoiding slime formation and/or for the reduction and/or removal of biofilm on surfaces of aqueous systems. In such process, the composition is either added to the aqueous system at a single location or at different points. If the enzymatic composition comprises more than one (active) component, it may be desirable under certain circumstances, to add the components at different sites.

The enzymatic composition may be added in an amount of 1 to 10,000 ppm, preferably 5 to 500 ppm of fermentation broth, relative to the system volume. According to a preferred embodiment of the present invention with regard to the colanic acid degrading activity, a concentration in the range of 0.001 to 100,000 units/ml, preferably 0.01 to 10,000 units/ml, is added.

The enzymatic composition of the present invention is suitable for slime prevention and biofilm removal in any aqueous system, i.e. either an open or closed industrial process-water system containing biofilm producing microorganisms. The use of the enzymatic composition of the invention is especially well suited for open or closed water cycles in paper factories, in particular white water cycles, or for cooling cycles. Further uses include biofilm removal in industrial cooling water towers, water storage tanks, water distribution systems, pulp and paper mill water as well as ultrafiltration and dialysis membranes and in health care. In addition it can be used in fermentation for control of viscosity, or for the production of specific mono or oligosaccharides.

According to the present invention it was particularly surprising that a single enzyme hydrolyzing (degrading) exopolysaccharides produced by *Enterobacteriaceae*, is suitable for prevention of biofilm formed by a wide variety of bacteria and for the reduction and/or removal of existing biofilm. Further, it was completely unexpected that this single enzyme, which is active against exopolysaccharides of a single microorganism exerts such high activity against exopolysaccharides in general.

The advantage of the present invention, amongst others, resides in the fact, that the enzyme develops its activity already at the very start of the biofouling process, in that the exopolysaccharidase prevents the adhesion of bacteria prior to biofilm formation. Additionally, the enzyme is capable of detaching already adhered bacteria and effectively removes biofilm from the surfaces of aqueous systems.

According to the invention, a synergistic or at least an additive effect with regard to slime reduction, removal and prevention of biofilm formation is achieved by combining the exopolysaccharidase with at least one further enzyme from the group consisting of polysaccharidases, proteases, lipases and glycoproteases, wherein proteases are most preferred.

The effect is especially enhanced by proteases such as serine endoprotease of *Bacillus licheniformis,* protease type XXI of *Streptomyces griseus,* or metalloprotease of *Bacillus polymyxa* type IX, and in particular by *Aspergillus* acid proteinase from *Aspergillus saitoi.*

According to the present invention, the enzyme and enzymatic compositions thereof are suitable for (a) preventing attachment of bacteria, (b) detaching adhered bacteria and/or (c) degradation of biofilm.

Although it is preferred to use the enzymatic composition or the exopolysaccharidase instead of biocides, combination with biocides is possible and may be desirable in certain cases.

The enzyme and/or enzymatic compositions of the present invention may also be combined with suitable surface-active agents such as surfactants and dispersants that would be readily apparent to those skilled in the art. Suitable surfactants may be selected from cationic, non-ionic, anionic, ampholytic and zwitterion surfactants. Examples of some of such suitable surfactants include, but are not limited to the following: suitable anionic surfactants include the water-soluble salts of alkyl benzene sulfonates, alkyl sulfates, alkyl polyethoxy ether sulfates, paraffin sulfonates, alpha-olefin sulfonates, alpha-sulfocarboxylates and their esters, alkyl glyceryl ether sulfonates, fatty acid monoglyceride sulfates and sulfonates, alkylphenol polyethoxy ether sulfates, 2-acyloxy-alkane-1-sulfonates, and beta-alkyloxy alkane sulfonates; suitable nonionic surfactants include water soluble ethoxylate materials such as fatty alcohol alkoxylates including fatty alcohol ethoxylates, primary and secondary ethoxylates, alkylphenol alkoxylates including alkylphenol ethoxylates, and EOPO (ethoxylate-propoxylate) block polymers.

The present invention is explained below with reference to examples.

In the examples, an enzyme capable of degrading exopolysaccharides (referred to as exopolysaccharidase or EPS'ase) is produced by fermentation of *Streptomyces* sp. ATCC 55601 in a nutrient medium followed by recovery of the enzyme from the broth. Underneath a detailed description is given of the respective strain *Streptomyces* sp., the production and recovery of the enzyme, the assay to determine the activity of an enzyme preparation and the application of the enzyme as an aid in the prevention and removal of biofilm.

Example 1

*Streptomyces* strain ATCC 55601 producing an enzyme capable of degrading exopolysaccharides, isolation and identification of the enzyme

1. Description of the strain

The strain ATCC 55601, producing the enzyme, was identified as belonging to the genus *Streptomyces* by 16S rRNA analysis and chemotaxonomic markers. A species identification could not be obtained by the data.

| Morphological markers | |
| --- | --- |
| Aerial mycelium | scant/grey |
| Spore chains | spiral |
| Melanin production | positive |
| Substrate mycelium | brown |
| Diffusible pigment | red |

| Chemotaxonomic markers | |
| --- | --- |
| LL-Diaminopimellic acid | present |
| Mycolic acid | absent |
| Menaquinone | MK-9 ($H_4$), MK-9 ($H_6$), MK-9 ($H_8$) |

Fatty acid profile: iso- and anteiso-branched fatty acids.

The microorganism is herein referred to as Streptomyces sp. It has been deposited at the American Type Culture Collection (ATCC), 12301 Parklane Drive, Rockville, Md. 20852 USA, under the Budapest Treaty on July 27, 1994, corresponding to ATCC designation 55601.

While *Streptomyces* sp. (ATCC 55601) comprises the preferred exopolysaccharidase source, other species of useful *Streptomyces* bacteria can be identified producing the exopolysaccharidase as described.

2. Growth of the strain and production of the enzyme

The strain (ATCC 55601) grows readily on different conventional media, i.e. for example Tryptic Soy agar. Aerial mycelium formation is limited to specific media like starch mineral salt agar. The enzyme can be produced by cultivating the microorganism *Streptomyces* sp. in a suitable culture medium containing a suitable carbon-source such as glucose, glycerol, lactose, fructose, sucrose, starch and the like. As nitrogen-source different substances can be used such as ammonium salts, nitrate salts and the like and/or complex organic nitrogen sources like peptones, yeast extract, meat extract, soybean extract, soybean meal, corn steep liquor, distillers dried solubles, casein, cottonseed protein and the like. Inorganic salts may include sodium chloride, potassium chloride, manganese chloride, sodium dihydrogen phosphate, potassium dihydrogen phosphate, disodium hydrogen phosphate, dipotassium hydrogen phosphate, magnesium sulfate, ferric sulfate, zinc sulfate, calcium chloride and the like. Furthermore organic stimulants may be added such as colanic acid, vitamins, soytone, corn steep liquor and the like. Additionally, antifoam may be added. Preferred culture media within the framework of the present invention are described for example by M. Sarra et al., Biotechnology Letters 15 (1993) 559-564.

The initial pH of the fermentation is preferably between 5.5 and 8. The pH may also be controlled during the fermentation, preferably between pH 5 and 9, more particularly between pH 5.5 and 8. Fermentation takes place between 20 and 40°C, preferably between 25 and 35°C.

A typical fermentation takes between 0.5 and 3 days, depending on the size of the inoculum and the fermentation parameters. Fermentation can be carried out as a conventional submerged fermentation or as a surface culture.

In addition to the direct production of the enzyme through a fermentation with *Streptomyces* sp. (ATCC 55601) the gene encoding said enzyme or fragments thereof may be cloned and expressed in a suitable expression vector. Techniques for the cloning of hydrolytic enzymes are well known to those skilled in the art and any suitable cloning procedure may be employed for the preparation of the exopolysaccharidase of this invention. Reference is made in this regard to J. Sambrook, E.F. Fritsch and T. Maniatis, "Molecular Cloning", Cold Spring Harbor Laboratory Press, 2nd Edition, 1989.

3. Recovery and downstream processing of the enzyme

After the fermentation, cells can be removed by centrifugation or filtration. If needed, the enzyme can be further concentrated and/or purified by techniques known in the art such as filtration, lyophilisation, extraction using aqueous two-phase systems or precipitation with organic solvents such as acetone and the like or inorganic salts such as ammonium sulfate. If needed chromatographic techniques such as gel permeation, ion exchange chromatography, affinity chromatography or other techniques known in the art can be used to purify and concentrate the supernatant broth. If no concentration factor is needed, the crude supernatant, preferably after cell removal, can be used as a raw source of enzyme.

4. Description of the enzyme assay

A. Whether the exopolysaccharidase is active or not can be assayed qualitatively via the method described underneath, further referred to as the spread plate (or "spot test") method.
   A strain of bacteria was grown in a liquid medium, e.g. trypticase soy broth, nutrient broth till logarithmic phase. Subsequently 100 µl of the culture was spotted on a solid medium, preferably with a high C/N ratio. Suitable media are described for example by M. Sarra et al., Biotechnology Letters 15 (1993) 559-564. The obtained culture was incubated for 2 or 3 days at 30°C until a uniform, slimy coverage of the medium occurred.
   Afterwards, 10 µl of exopolysaccharidase was added on top of the slimy layer. If clearing holes (i.e a "halo") appeared, the enzyme was considered as being active against the strain under evaluation.

B. The activity of the enzyme can also be assayed quantitatively by a viscometric assay. If the enzyme is active against the test sample of bacteria, slime and/or polysaccharides produced by them, then the addition of enzyme to such test sample will result in a reduction of viscosity, due to the enzymatic hydrolysis of the polysaccharides.

A solution of the expolysaccharides (hereinafter also referred to as EPS) was made (by the method of Example 2, as follows) and incubated with a certain amount of enzyme. Afterwards the mixture was heated at 100°C during ten minutes to inactivate the enzyme. Subsequently the inactivated mixture was diluted with distilled water to a total volume of 16 ml.

Viscosity was subsequently determined using an Übbelohde viscometer. One unit of activity was taken as that amount of enzyme causing, under the given conditions, i.e. pH 7 and 30°C, a viscosity reduction of 50% in 30 min.

Viscosity reduction was determined according to the following formula:

$$\text{Viscosity reduction (\%)} = \frac{\text{(Viscosity Blank 1-Viscosity sample)}}{\text{(Viscosity Blank 1-Viscosity Blank 2)}} \times 100$$

whereby:

Viscosity Blank 1:     Enzyme was inactivated prior to addition to the EPS-solution
Viscosity Blank 2:     Enzyme was added to a solution containing 3 ml of buffer instead of EPS-solution.

### 5. Characteristics of the exopolysaccharidase produced by ATCC 55601

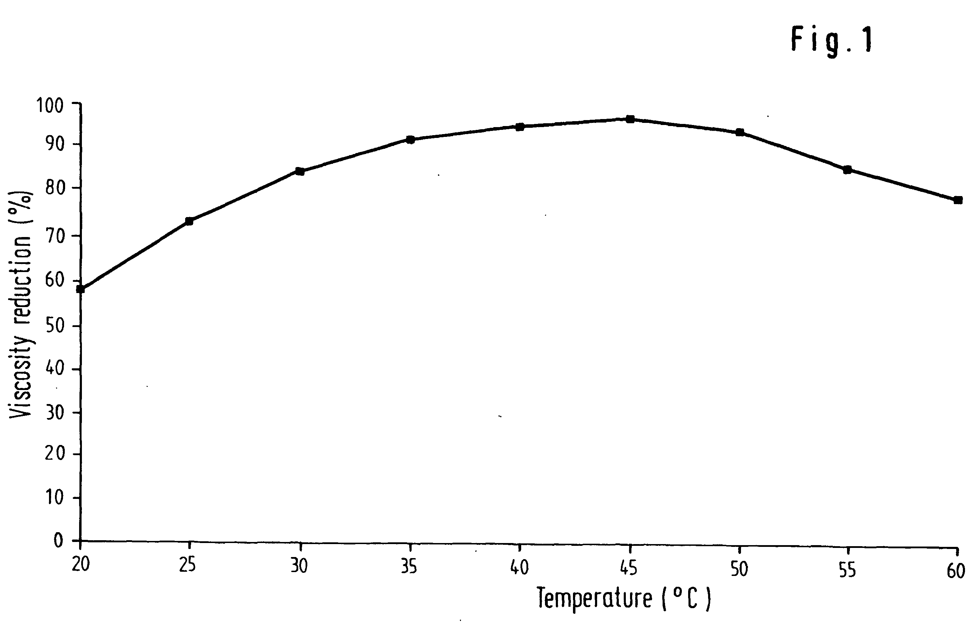

The exopolysaccharidase is an extracellular enzyme with a molecular weight of about 100 kDa, as determined by SDS-PAGE under denaturing conditions. Activity as a function of temperature is given in Fig. 1. Highest activity is seen around 45°C.

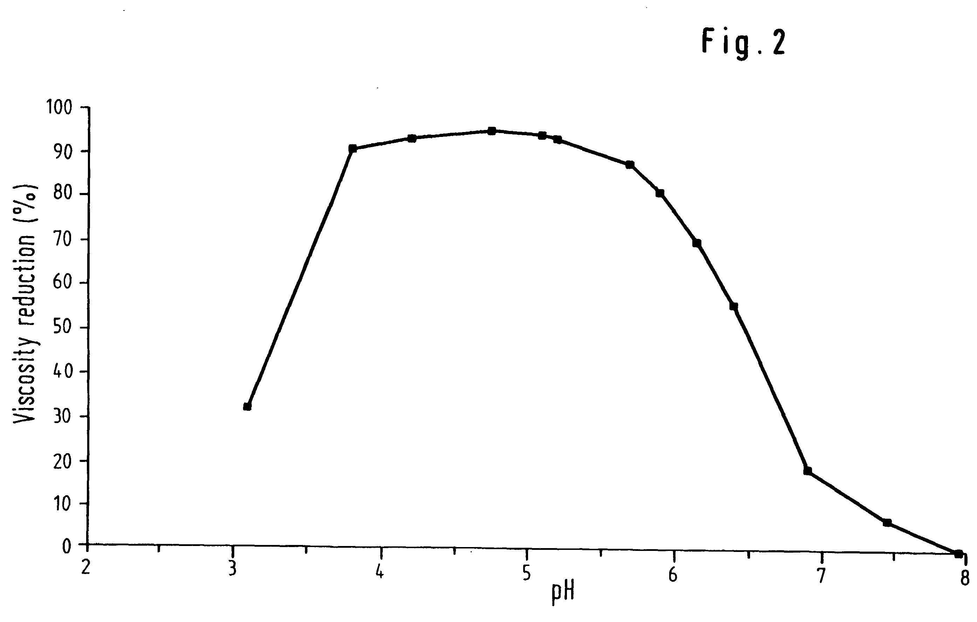

Fig. 2 shows the activity as a function of the pH. The optimum pH is situated between pH 4.5 and 5.5.

Thermostability studies on the native enzyme indicate a half life time at 35°C of 3.5 h, and at 50°C of 45 minutes. This thermostability can be enhanced by methods known in the art such as the addition of glycerol, sorbitol, glucose, EDTA etc.

The enzyme is not inactivated by proteases such as serine endo protease of *Bacillus licheniformis* (e.g., Alcalase® - Novo, Esperase® - Novo) protease type XXI of *Streptomyces griseus,* metalloprotease of *Bacillus polymyxa* type IX. In combination with *Aspergillus* acid proteinase (from *Aspergillus saitoi*) an even more active preparation is obtained. The combination of EPS'ase with *Aspergillus* acid proteinase influences the activity (with regard to prevention of bacterial adhesion, detachment of adhered bacteria and/or biofilm removal) on scales between 1/99 to 99/1 EPS'ase/ proteinase.

It is beneficial to combine these proteases and the like with the exopolysaccharidase for the use of slime control. Other proteases suitable for slime removal are well known from the art, for example from EP 0 590 746.

### 6. Activities of the supernatant broth from cultivation of ATCC 55601

Besides the specific exopolysaccharidase activity, the following activities were detected, using p-nitrophenyl derivates of the corresponding sugars:

*     N-acetyl-β-D-glucosaminidase
*     Alpha-L-fucosidase

Using sulfanilamide-azocasein (R.M. Tomarelli et al., J. Lab. Clin. Med. 34, 1949, 428) protease acticvity was also observed.

The observed activities can advantageously contribute to the biofilm prevention and/or removal.

When the enzyme was spotted on a cell layer consisting of the following culture collection strains, a clear effect was visible:

    *Enterobacter cloacae* NCTC 9395
    *Klebsiella oxytoca* LMG 3055
    *Klebsiella terrigena* LMG 3207
    *Klebsiella planticola LMG* 3065
    *Klebsiella pneumoniae LMG* 2095
    *Pseudomonas picketii* LMG 5942
    *Xanthomonas campestris* NRRL 1453.

The activity of the EPS'ase against various bacteria of aqueous systems was confirmed by using the qualitative enzyme assay (spot test of 4.A., above) with bacteria isolated from slime samples. The bacteria were grown on potato Dextrose Agar, and incubated for 48 hours at 30°C to form a cofluent layer of slime. A 3 % stock solution of EPS'ase was prepared. Then, 10 μl of 3 % stock solution of EPS'ase was spotted on the layer. The results were as follows:

| Sample No. | Bacterium | Result |
|---|---|---|
| 73 | *Enterobacter sp.* | Positive-Clearing |
| 74 | *Klebsiella sp.* | Positive-Clearing |
| 84 | Not identified | Positive-Clearing |
| 86 | Not identified | Positive-Clearing |
| 87 | *Ocrobacterium antropi* | Negative |
| 88 | *Achrobacterium radiobacter B* | Positive-Clearing |
| 91 | *Pseudomonas foragi* | Positive-Clearing |

This experiment demonstrates that the EPS'ase of the invention is not only active against *Enterobacteriaceae* but also against other bacterial species (i.e., *Achrobacterium* and *Pseudomonas).* However, activity against the *Enterobacter* strain is far more pronounced than against the other strains.

The EPS'ase of the present invention may also be used in combination with other commercially available enzymes. The above spot tests were repeated using EPS'ase (in a 3% stock solution, denoted heirein as "EPS'ase 3%) with three commercial enzymes, Esperase® , Cereflo® , and Gamanase® , obtained from Novo Nordisk.

Esperase® is a serine-type protease produced by submerged fermentation of an alkalophilic species of *Bacillus.*

Cereflo® is a purified bacterial β-glucanase preparation produced by submerged fermentation of *Bacillus subtilis;* the enzyme os an endo-glucanase which breaks down malt and barley β-glucan (1,4-β-,-1,3-β-glucans) to oligosaccharides with 3-5 glucose units.

Gamanase™ is a galacto-mannanase (1,4-β-D-Mannan mannanohydrolase) prepared from *Aspergillus niger;* the enzyme randomly hydrolyzes β (1-4) bands in mannans, galactomannans, and glucomannans.

In this test, the following combinations were used on the bacterial isolates. The enzymes were mixed together in a 50:50 ratio. Ten μl of each mixture was spotted on the bacterial layer.

   (a) EPS'ase 3% + Esperase® 8.0L (50/50)
   (b) EPS'ase 3% + Cereflo® 200L (50/50)
   (c) EPS'ase 3% + Gamanase™ 1.5L (50/50)

The results were as follows:

| Sample No. | Bacterium | Positive Result |
|---|---|---|
| 73 | *Enterobacter sp* | a, b, c |
| 74 | *Klebsiella sp* | a, c |
| 84 | Not identified | b, c |
| 86 | Not identified | a, b, c |
| 87 | *Ocrobacterium antropi* | a, c |
| 91 | *Pseudomonas fragi* | a, b, c |

The above result provide substantial evidence that the EPS'ase of the present invention may be suitably used in combination with other, commercially available, enzymes.

7. Biofilm prevention and removal capabilities

Biofilm formation does take place in different environments such as in industrial cooling water towers, water storage tanks, water distribution systems, pulp and paper mill water, ultrafiltration and dialysis membranes in health care.

In experiments simulating the process water of a paper mill (see below), the application of the exopolysaccharidase, subject of this invention, reduced the thickness of the biofilm compared to a control, by hydrolyzing the slime that holds the biofilm together. The application of this enzyme reduces the formation of biofilm through two mechanisms, by preventing the attachment of bacteria and by promoting the detachment of adhered bacteria.

The amount of exopolysaccharidase needed to prevent or to remove biofilm depends on the contamination of the system. As a guideline, dosages between 1 and 10,000 ppm, preferably 5 to 500 ppm, are appropriate. With regard to colanic acid degrading activity, dosages between 0.001 and 100,000 units/ml, preferably 0.01 to 10,000 units/ml, are appropriate. The enzyme can be slug (shock) or pulse dosed or - if needed - continuously added. As it is easier to dose liquid formulations, a liquid composition comprising said enzyme is preferred. Where needed, a dry product may be added, such as in tablet form. If added as a liquid formulation, the enzyme is preferably stabilised according to the methods known in the art, such as by the addition of glucose, sorbitol, glycerol, EDTA etc.

## Example 2

A representative exopolysaccharide structure, or "EPS" structure, for quantitative assays can be made according to the method of Garegg et al. (Acta Chem. Scand. 1971, 25(4), 1185-1194). A bacterial strain, preferentially *Enterobacter cloacae* NCTC 9395, was grown on a solid medium (pH 7) with the following composition:

| Component | Concentration (g/l) |
|---|---|
| D-Glucose | 4.0 |
| $NH_4Cl$ | 0.4 |
| $Na_2HPO_4 \cdot 2H_2O$ | 7.5 |
| $KH_2PO_4$ | 3.0 |
| NaCl | 0.5 |
| $MgSO_4 \cdot 7H_2O$ | 0.2 |
| Agar | 15 |

The bacteria were grown for 48 hours at 30°C. Subsequently they were scraped off and suspended in phosphate buffer, pH 7. Afterwards the solution was centrifuged for 30 min at 30000 G. The supernatant was dialyzed aginst distilled water and brought to pH 10-11 by addition of 0.1 N NaOH. EPS present in this preparation was precipitated by addition of two volumes cold acetone (-18°C). The obtained EPS was then freeze-dried.

## Example 3

The *Streptomyces* sp. strain ATCC 55601 was grown in an erlenmeyer flask with the following medium (pH 7):

| Component | Concentration (g/l) |
|---|---|
| Yeast Extract | 10 |
| Starch | 10 |
| Tryptone | 5 |
| $MgSO_4 \cdot 7H_2O$ | 0.5 |
| $MnCl_2 \cdot 4H_2O$ | 0.1 |
| EPS | 2 |

The culture was grown for 36 hours on a rotary shaker at 250 rpm and 30°C. Activity was assayed via a spot-test against *Enterobacter cloacae* NCTC 9395. Clearing holes appeared within one hour, indicating a high exopolysaccharidase activity. Afterwards the broth was centrifuged and the superatnatant was brought to 65% saturation with ammonium-sulfate. The precipitate was freeze-dried and used as a crude source of exopolysaccharidase (EPS'ase).

## Example 4

Two g of the lyophilized EPS'ase powder as described in Example 3 were dissolved in 100 ml Tris buffer 0.1 M, pH 7. *Enterobacteriaceae* EPS (0.5 g) was dissolved in 100 ml Tris buffer (0.1 M, pH 7). 1 ml of the EPS'ase was combined with 3 ml of the EPS solution and incubated for 30 min at 30°C. After 30 min, the reaction was stopped by heating the mixture for 5 min at 100°C. A first blank contained 1 ml of previously inactivated enzyme with 3 ml EPS. The second blank contained also 1 ml inactivated enzyme but the EPS solution was replaced by 3 ml of Tris-buffer. Viscosity of the three samples was determined with an Übbelohde viscometer at 30°C with an automatic registration. Results are given in Table 1.

Table 1:

| Viscosities of the samples (pH=7, Temp.=30°C) | |
|---|---|
| Sample | Viscosity ($mm^2$/s) |
| Viscosity first blank (EPS + inactivated EPS'ase | 3.079 |
| Viscosity second blank (Buffer + inactivated EPS'ase | 0.827 |
| Viscosity sample (EPS + EPS'ase) | 1.010 |

The exopolysaccharidase causes a distinct decrease in viscosity of the EPS-solution, which demonstates degradation of exopolysaccharides. Calculated by the formula of Example 1, 4.B, this viscosity reduction was 91.9%.

Example 5

To test the removal of biofilm, a biofouling reactor was used, simulating the wet-end section of a paper mill. The bacterium grown in this fermentor was a troublesome isolate of a paper mill, belonging to the *Enterobacteriaceae* and known to produce large amounts of EPS. In the mixing vessel, the bacteria were mixed with the dilution water and nutrients. The following concentrations of the nutrients were applied in the test unit:

| Compound | Concentration (ppm) |
|---|---|
| NaCl | 10 |
| $K_2HPO_4$ | 3 |
| Glucose | 200 |
| Xylose | 100 |
| Galactose | 20 |
| Arabinose | 1 |
| Mannose | 0.5 |
| Fructose | 20 |
| Cationic starch | 30 |
| Bacto Soytone | 20 |

This mixing vessel was connected to PVC ducts that were equipped with circular metal test sections. These sections were weighed before and after the enzyme addition. Also the total suspended solids were monitored during the addition of the enzyme.

The unit was operated at a temperature between 30 and 35°C, pH 7 and the mean residence time was one hour.

The biofilm was allowed to develop for seven days. After this period a biofilm of 2-5 mm thickness had developed. As enzyme, a crude supernatant of an erlenmeyer culture of *Streptomyces* sp. was used. Enzyme was added in four equal parts in shock doses.

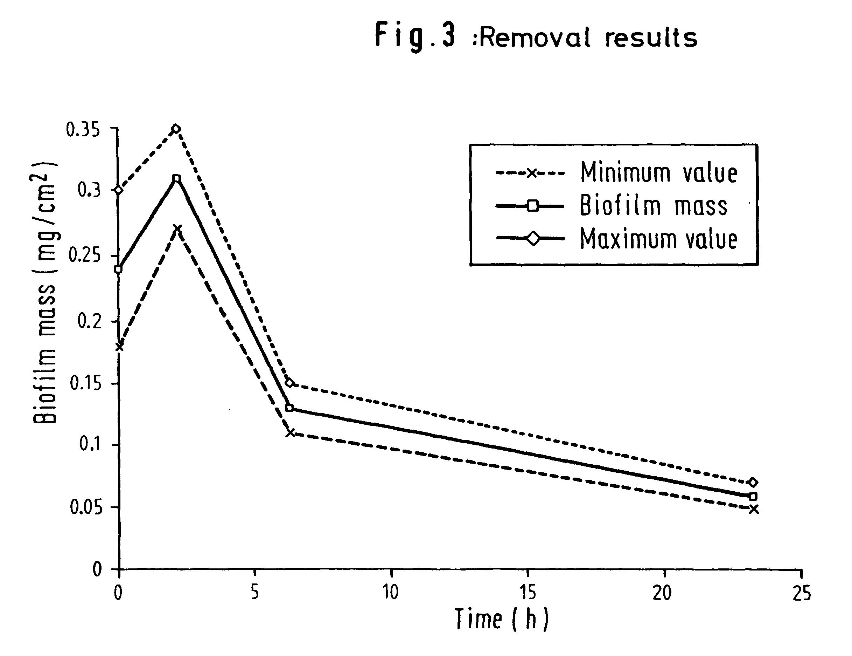

Each shock dose corresponded to a dose of 4500 ppm. Gravimetric determinations are given in Fig. 3.

24 hours after the beginning of the addition 75% of the biofilm was removed. Simultaneously with the addition, the removal could also be followed visually. Larger parts of biofilm, formed in the mixing vessel, sloughed upon the addition of enzyme. This removal did not take place continuously but stepwise, probably due to the high amounts of slime that had formed in the rig.

Example 6

The prevention capabilities of EPS'ase were evaluated with a second rig system, equipped with IR detectors. IR absorption is linearly correlated to biofilm thickness. The rig was operated at the following conditions:

| Temperature | 35°C |
|---|---|
| pH | 7 |
| Mean Residence Time | 20 min |
| Flow velocity | 0.66 m/s |

The isolate from a paper mill was again used as inoculum (cf. Example 5). Bacterial concentration in the rig system was initially set to $5.0 \cdot 10^6$ CFU/ml.

EPS'ase was dosed four times a day during 30 minutes. Highest concentration in the system was 200 ppm after 30 min.

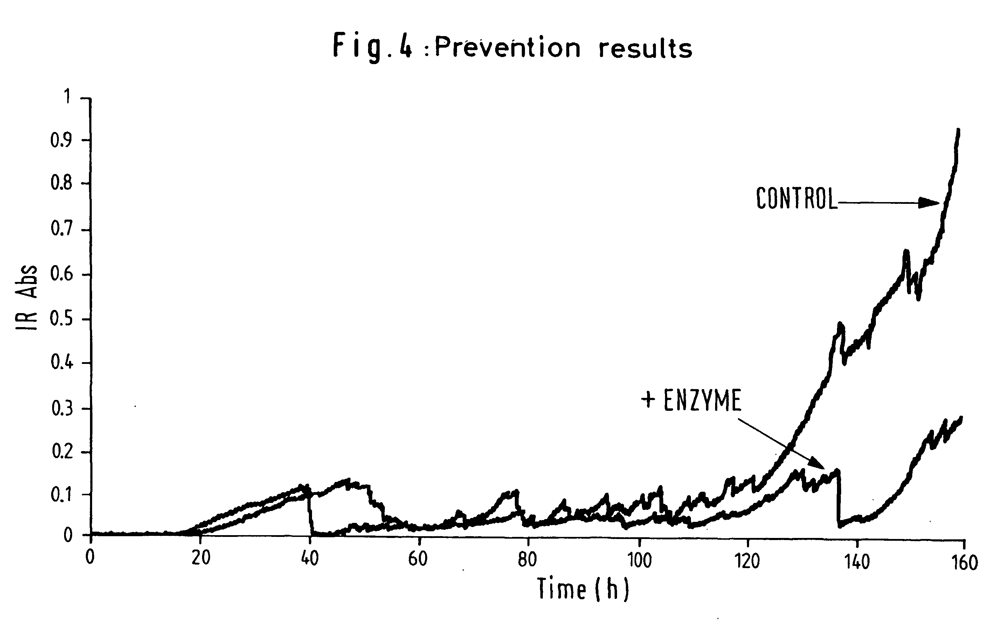

Biofilm formation in the enzyme treated rig was far below the development in the untreated rig (Fig. 4). Both rigs were run simultaneously. This demonstrates that the exopolysaccharidase is not only useful in the removal of bioslime but also in its prevention.

Example 7

In order to verify whether the observed effect of biofilm removal and prevention was solely due to an enzymatic hydrolysis of EPS the following experiment was carried out. A bacterial isolate from a paper mill was grown in a liquid medium (pH 7) with the following composition:

| Component | Concentration (g/l) |
|---|---|
| Glucose | 15 |
| Bacteriological Peptone | 7 |
| NaCl | 5 |

Two erlenmeyers of 500 ml were filled with 100 ml of this medium and inoculated with the bacteria. To one erlenmeyer 1 ml of EPS'ase solution was added. Growth in both erlenmeyers was followed by measuring the optical density at 600 nm. At the end of the fermentation, after 30 hours, the content of the erlenmeyers was centrifuged and the viscosity of the supernatant was determined (Table 2).

Table 2:

| Viscosity of treated and untreated erlenmeyer flasks after 30 hours fermentation. | |
|---|---|
| Erlenmeyer flasks | Viscosity ($mm^2$/s) |
| Untreated | 3.977 |
| + Enzyme | 1.212 |
| Sterile both | 0.834 |

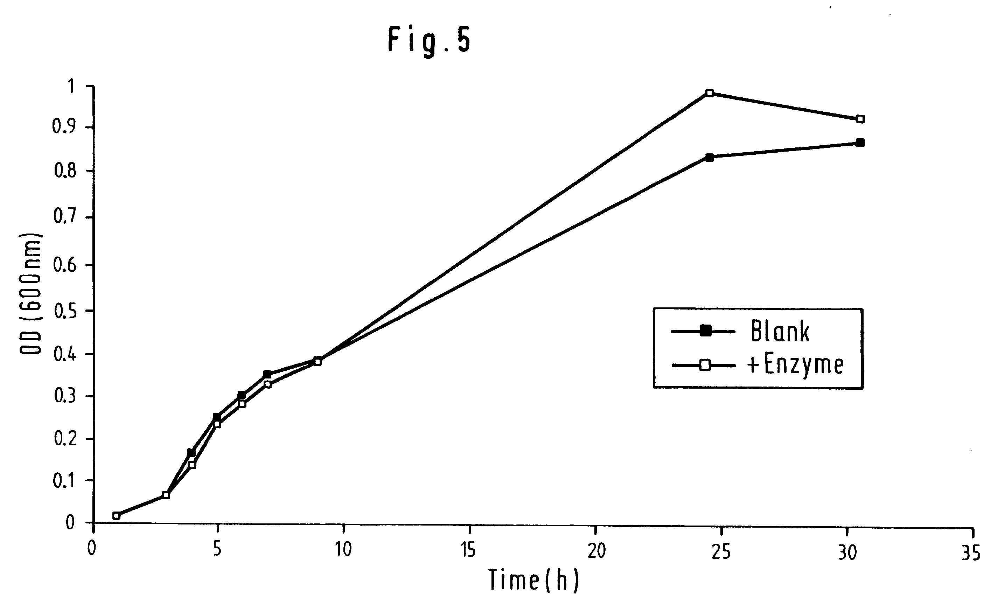

The formation of EPS is prevented by the administration of EPS'ase. Growth is not affected as the optical density in both erlenmeyers gave comparable results (Fig. 5).

This experiment proves that the supernatant of *Streptomyces* sp. exerts only an effect on the EPS and that the enzyme preparation does not kill or limit bacterial growth.

The above experiment further proves that the enzyme could be used to control rheology and viscosity parameters in the fermentation of EPS-forming microorganisms. It can be applied in fermentations where exopolysaccharides are produced with the aim to control viscosity. This would enhance the oxygen transfer in the fermentor and reduce energy consumption for stirring purposes.

Furthermore, the exopolysaccharidase can be used in isolated (i.e. purified) form. The isolation can be conducted by suitable methods for the recovery and purification of enzymes well known in the art.

Additionally, the enzyme can be used to hydrolyse polysaccharides after production instead of acid hydrolysis.

An example application for this is the intentional separation of sugars from polysaccharides. For example, the polysaccharide can be colanic acid.

Example 8

The EPS-forming organism (cf. Example 7) was allowed to produce EPS. Then the culture was centrifuged and resuspended in synthetic mill buffer.

It is known that a lot of enzymes can be inactivated by biocides, so each experiment was divided into two parts. Apart from a biocide control where no enzyme was present and a growth control, there was a set where the enzyme and the biocide were added at the same time (simultaneous experiment) and another set where the biocide was added one hour after the enzyme (sequential experiment). In that way, the enzyme had the chance of breaking down the EPS that was formed during the growth of the culture, before the biocide was added. In Table 3 results are shown for Bronopol (2-bromo-2-nitro-1,3-propandiol), demonstrating a positive effect. The concentration necessary for a 99.99% kill after 3 hours is halved (i.e. from 25 to 12.5 ppm) when the enzyme is applied before the biocide. Also shown are the results when using Kathon WT (5-chloro-2-methyl-4-isothiazolin-3-one + 2-methyl-4-isothiazolin-3-one, 13.9 %) and glutaraldehyde.

EP 0 820 516 B1

## Table 3 :

### Influence of EPS'ase on biocidal activity

| Biocide Control | | | | | | | | |
|---|---|---|---|---|---|---|---|---|
| Time | Control | Dilution | K12,5ppm | K25ppm | B12,5ppm | B25ppm | G200ppm | G400ppm |
| 30' | +31,11 | 1/100 | +2,22 | -10 | +2,22 | -2,22 | -27,77 | ? |
| | | 0 | ? | ? | ? | ? | ? | -99,99 |
| 180' | +694,44 | 0 | ? | -99,98 | ? | -99,99 | -99,92 | -99,99 |
| Simultaneous experiment | | | | | | | | |
| Time | Control | Dilution | K12,5ppmE | K25ppmE | B12,5ppmE | B25ppmE | G200ppmE | G400ppmE |
| 30' | +70,59 | 1/100 | +47,06 | +45,59 | +52,94 | +45,59 | -13,24 | ? |
| | | 0 | ? | ? | ? | ? | ? | -99,99 |
| 180' | +1032,35 | 0 | ? | -98,49 | ? | -99,99 | ? | -99,99 |
| Sequential experiment | | | | | | | | |
| Time | Control | Dilution | K12,5ppmS | K25ppmS | B12,5ppmS | B25ppmS | G200ppmS | G400ppmS |
| 30' | +19,77 | 1/100 | +24,42 | +6,98 | -5,81 | -31,40 | -11,63 | -98,35 |
| | | 0 | ? | ? | ? | ? | ? | -99,99 |
| 180' | +795,35 | 0 | ? | -99,95 | -99,81 | -99,99 | ? | 98,49 |

' = Time in minutes
- % = % kill compared to growth control at t = 0'
+ % = % growth compared to growth control at t = 0'
? means that plates are too much overgrown to count
K = Kathon WT, B = Bronopol, G = glutaraldehyde

Example 9

To further demonstrate the activity of the EPS'ase with respect to various bacteria, and the slime produced by them, *Acinetobacter* sp. was tested using the viscometric assay (see Example 1, 4.B). *Enterobacter* sp. was simultaneously tested for comparison.

The specific samples chosen for analysis were selected based upon their large plate counts of single cell bacteria (determined by microscopy), and those which further had a slimy matrix. The *Acinetobacter* sp. was identified as comprising *Acinetobacter baumannii/genospecies 2* including *Pseudomonas mendocina, Pseudomonas fluorescens C, Pseudomonas putida A* and *Acinetobacter calcoaceticus/genospecies* 2. The *Enterobacter* sp. was identified as comprising *Klebsiella pneumoniae* A including *Klebsiella terrigena.*

Biofilm was produced for both *Acinetobacter* sp. and *Enterobacter* sp. using the biofouling reactor rig of Example 5. The biofilm slimes were then isolated from the walls of circular metal test sections, and each subjected to the same high speed centrifugation to separate out the respective exopolysaccharide solutions. To samples of each exopolysaccharide solution, a 3% stock solution of EPS'ase ("EPS'ase 3%") was added at a concentration of 1000 ppm, and then incubated for 2 hours at 35°C and 50 rpm. Decrease in viscosity was calculated (by the method of Example 1, 4.B) taking into account a heat-inactivated control and water. The results were as follows:

| Test Sample | % Change in Viscosity |
| --- | --- |
| *Acinetobacter* sp. + EPS'ase 3% | -19 |
| *Enterobacter* sp. + EPS'ase 3% | -32 |

These results demonstrate that the EPS'ase of the present invention is not only active against *Enterobacteriaceae,* but also against other bacteria commonly found in water systems.

Example 10

Exopolysaccharide (EPS) was purified from a bacterial isolate (*Enterobacter*) from a paper mill by growing the microorganism in a suitable medium that favored EPS formation, collecting the EPS from the medium by acetone precipitation and freeze-drying the final product.

*Enterobacter* was grown in 2-liter fermenters containing a working volume of 1.2 liters of the following medium (in g or ml per liter): Bacto-Peptone, 7 g; NaCl, 5 g; $K_2HPO_4$, 3 g; $KH_2PO_4$, 0.9 g; Glucose, 15 g, and antifoam 8270 (Dearborn Chemicals), 0.05 ml. Fermentations were performed at 30°C, pH 7.0, with an agitation of 750 rpm and were aerated with 0.6 volume of air per volume of medium per minute. After 56 hours, the fermentation broth was centrifuged at 13,600 X g for 1 hour to remove cells. One liter of ice-cold acetone was added to the culture supernatant fraction (1900 ml). This resulted in gelatinous material that floated on the surface of the mixture. The gelatinous material was skimmed from the surface, and the pH of the remaining solution was adjusted to pH 11 by the addition of 1 N NaOH. An additional 500 ml of acetone was added, and the solution was chilled for 24 hours at 4°C. This resulted in additional gelatinous material that floated on the surface of the solution. This material was collected from the surface and combined with the first gelatinous fraction. The combined fractions were dissolved in Milli-Q water overnight at 4°C then dialyzed exhaustively against Milli-Q water until the conductivity of the dialysate was 0.037 microOhms/cm$^2$. Following dialysis, the dialysate was lyophilized, and stored at 4°C.

Example 11

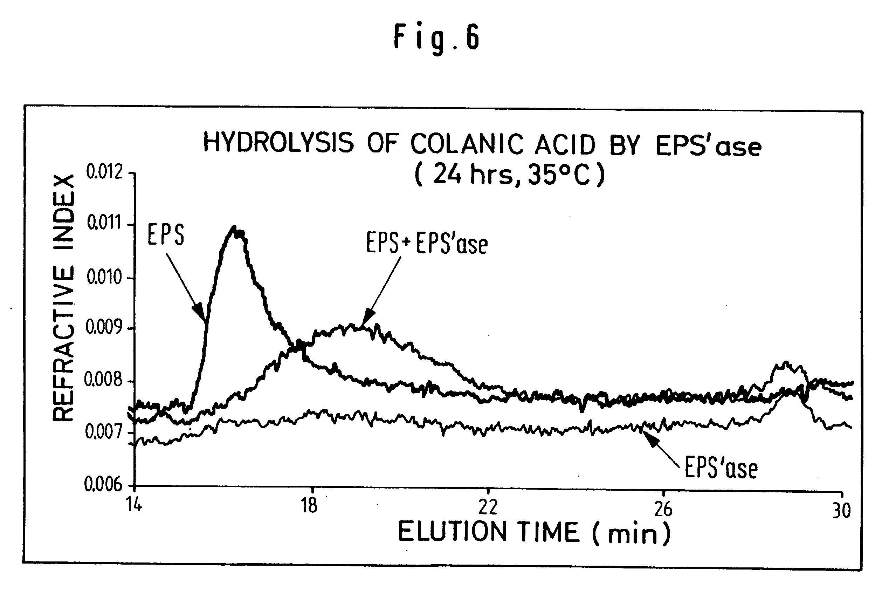

Gel permeation chromatography shows that the *Enterobacter* EPS prepared in Example 10 has a MW of above $2.10^6$. No other molecular weight fraction is observed in the $10^4$ - $10^6$ region. After 3 hours of contact time using EPS'ase, no degradation products are visible; although viscosity has already decreased by then substantially. After 24 hours, the peak at MW>$2.10^6$ has disappeared and a new broad peak is observed at $4.10^5$. This remains essentially constant at even longer incubation times. Degradation of purified EPS by EPS'ase as shown by GPC is shown in Fig. 6.

Example 12

Glycosyl composition analysis of the EPS prepared according to Example 10 was performed by the preparation and GC analysis of alditol acetates both before and after carboxyl group reduction.

The methods are described by York, et al. (*Methods Enzymol.* 118 (1985) 3-40). Briefly, a small amount of the *Enterobacter* EPS was hydrolyzed using 2 M trifluoroacetic acid (TFA) at 121° for two hours. The resulting monosaccharides were reduced to their alditols, using sodium borodeuteride. The resulting alditols were then acetylated, using acetic anhydride in pyridine. The alditol acetates were analyzed by GC, using a capillary SP2330 column from Supelco. In order to characterize the type of uronic acid that was present, a small amount of sample was treated with methanolic 1 M HCl at 80° for a few hours. The resulting methylglycosides, which would include the methyl ester of any acidic sugar such as glucuronic or galacturonic acid, were reduced using sodium borodeuteride. This procedure reduces the methyl esters of uronic acid to the corresponding hexose. For example, glucuronic acid methyl ester will be reduced to glucose. The resulting mixture was then hydrolyzed with TFA and converted to alditol acetates as described above. GC analysis is also performed as described above.

Glycosyl linkage analysis was performed by preparing and analyzing partially methylated alditol acetates. This was done by the procedure of Ciucanu and Kerek (*Carbohydrate Res.,* 131 (1984) 209-217). In this method, the sample was dissolved in dimethyl sulfoxide (DMSO) and powdered sodium hydroxide was added to produce the DMSO anion. This was stirred for several hours at room temperature after which an excess of methyl iodide was added to form the permethylated polysaccharide. At this point the permethylated sample was divided into two parts. One part was hydrolyzed in TFA and converted to the alditol acetates as described above. The second part the polysaccharide was methanolyzed in 1 M HCl in methanol and the resulting partially methylated methyl glycosides were treated with sodium borodeuteride in order to convert any partially methylated uronic acids residues into their corresponding hexoses. When this conversion is made, these sugar residues will then have two deuterium atoms attached to C-6 which enables them to be distinguished from normal hexoses on the mass spectrometric analysis. The resulting permethylated carboxyl reduced methyl glycosides were then hydrolyzed and converted to partially methylated acetates as described above. Analysis of both parts was done by combined GC-MS using an SP2330 capillary column from Supelco.

**Results:**

Glycosyl composition and methylation results are shown in Table 4 below:

Table 4:

| Glycosyl Composition Analysis of *Enterobacter* EPS prepared in Example 10 | |
|---|---|
| Glycosyl Residue | Relative Percent |
| Fucose | 37 |
| Galactose | 34 |
| Glucose | 29 |

For the composition given in Table 4, the analysis was done prior to carboxyl group reduction. After carboxyl group reduction, the amount of glucose significantly increased showing the type of uronic acid residue present in this EPS was glucuronic acid.

Table 5 shows the results of glycosyl linkage analysis of this extracellular polysaccharide.

Table 5 -

| Glycosyl Linkage Analysis | |
|---|---|
| Glycosyl Residue | Relative GC Peak Area % |
| Terminal linked galactose | 4.1 |
| 4-linked fucose | 14.0 |
| 3,4-linked fucose | 20.0 |
| 3-linked glucose | 20.0 |
| 3-linked galactose | 19.0 |
| 4,6-linked galactose | 22.0 |

The linkage analysis after carboxyl group reduction results in an additional peak, identified as 4-linked glucuronic acid which was present in equal amounts to 4,6-linked galactose, etc. These results indicate that this EPS has a 1:1:1:1:1:1 ratio of 4-linked fucose:3,4-linked fucose:3-linked glucose:3-linked galactose:4-linked glucuronic acid: 4,6-linked galactose. These linkages are generally consistent with previous published structures for colanic acid (cf. G. Aspinall (ed.) "The Polysaccharides", vol. II (1983), Academic Press).

Example 13

Additional rig studies were carried out confirming that EPS'ase prevents biofouling by *Enterobacteriaceae* (cf. Example 6). The EPS'ase used for these rig studies was obtained according to Example 3.

Biofilm prevention capablities were evaluated utilizing the rig described in Example 5:

| Test conditions: | |
|---|---|
| flow velocity | 0.74 m/s |
| Temperature | 35°C |
| pH | 7 |
| Mean residence time | 1 hour |
| strain used to foul rig: | *Enterobacter* sp. isolate from field slime (cf. Example 5) |
| duration of test | 100 hours |

**Test A**

Dosing regime for EPS'ase: 4 times per day 30 minutes resulting in peak concentration of (94 ppm) $4.4 \cdot 10^5$ units of colanic acid degrading activity/l. This resulted 35% inhibition.

**Test B**

Dosing regime for EPS'ase: 4 times per day 30 minutes resulting in peak concentration of 44 units of colanic acid degrading activity/l. This resulted in 30% inhibition.

Activity units of EPS'ase for rig studies was determined as follows:

1. From an EPS fermentation broth, serial dilutions in 50 mM MES(pH 5.5) (MES = 2-(N-Morpholino)ethanesulfonic acid) were prepared.
2. 25 µl from each dilution were spotted onto an 48 hour old *Enterobacter* lawn surface. Incubation took place for 60 minutes at 32°C.
3. Clear zone diameters were measured.
4. Each clear zone diameter was multiplied with the corresponding dilution level.
5. The sum of all the products equals the EPS'ase units/0.025 ml.

Example 14

The following experiment provides additional proof for the fact that EPS'ase is an exopolysaccharide degrading enzyme, i.e. EPS'ase activity is not restricted to *Enterobacteriaceae.*

The experiment described in Example 1, point 6, above was repeated using the identified bacteria isolated from field slimes, listed below. Halo's were visible on cell layers of the following isolates. Application of inactivated enzyme did not result in halo's.

*Enterobacter* sp. isolate 1
*Enterobacter* sp. isolate 2
*Pseudomonas fragi*
*Klebsiella pneumoniae A*
*Pseudomonas mendocina*
*Pseudomonas fluorescens*
*Enterobacter asburiae*

**Description of the Figures:**

Fig. 1 shows the activity of the enzyme of Example 1 as a function of the temperature.

Fig. 2 shows the activity of the enzyme of Example as a in function of the pH. The optimum is situated between pH 4.5 and 5.5.

Fig. 3 shows the results for biofilm removal as a function of the time using the enzyme of Example 1 (cf.

Example 5).

Fig. 4 shows the results for slime prevention as a function of the time using the enzyme of Example 1 (cf. Example 6).

Fig. 5 shows the results for prevention of EPS formation as a function of the time using the enzyme of Example 1 (cf. Example 7).

Fig. 6 shows degradation of purified EPS by EPS'ase as determined by GPC (cf. Example 11).

## Claims

1. Exopolysaccharide degrading enzyme, obtainable by cultivating in a suitable medium the *Streptomyces* strain ATCC 55601, **characterized in that** it has a molecular weight of about 100 kDa, as determined by SDS-PAGE under denaturing conditions, and is capable of degrading colanic acid.

2. Enzymatic composition for the reduction, removal and/or prevention of biofilm on surfaces of aqueous systems, **characterized in that** it comprises the exopolysaccharide degrading enzyme of claim 1.

3. Composition according to claim 2, **characterized in that** the composition further comprises at least one enzyme from the group consisting of polysaccharidases, proteases, lipases and glycoproteases.

4. Composition according to claim 3, **characterized in that** the enzyme is a protease selected from the group consisting of serine endoprotease produced by *Bacillus licheniformis,* protease type XXI of *Streptomyces griseus,* metalloprotease of *Bacillus polymyxa* type IX, and *Aspergillus* acid proteinase of *Aspergillus saitoi.*

5. Process for the production of the enzyme of claim 1, **characterized in that** the *Streptomyces* strain ATCC 55601 is cultivated in a suitable medium.

6. Process for the production of the enzyme of claim 1, **characterized in that** a mutant, a variant or a transformant of the *Streptomyces* strain ATCC 55601, which produces the enzyme of claim 1, is cultivated in a suitable medium.

7. Use of the enzyme of claims 1 and/or of the composition of claims 2 to 4 for the reduction, removal and/or prevention of biofilm on surfaces of aqueous systems, wherein the use is not for treatment of the human or animal body by therapy practised on the human or animal body.

8. Use according to claim 7, **characterized in that** the aqueous system is an open or closed industrial process-water or cooling-water system.

9. Use according to claim 8, **characterized in that** the industrial process-water system is an open or closed water cycle in a paper factory.

10. Use according to claim 7, **characterized in that** the surfaces are ultrafiltration or dialysis membranes.

11. Use of the enzyme of claim 1 and/or the composition of claims 2 to 4 for the degradation of exopolysaccharides, wherein the use is not for treatment of the human or animal body by therapy practised on the human or animal body.

12. *Streptomyces* strain ATCC 55601.

13. Mutant, variant or transformant of the *Streptomyces* strain of claim 12, which produces the enzyme of claim 1.

## Patentansprüche

1. Exopolysaccharid-abbauendes Enzym, das durch Kultivierung des *Streptomyces-Stamms* ATCC 55601 in einem geeigneten Medium erhältlich ist, **dadurch gekennzeichnet, dass** es ein Molekulargewicht von etwa 100 kDa aufweist, das durch SDS-PAGE unter denaturierenden Bedingungen bestimmt ist, und welches in der Lage ist, Colansäure abzubauen.

2. Enzymatische Zusammensetzung zur Reduktion, Entfernung und/oder Verhinderung eines Biofilms auf Oberflächen wässriger Systeme, **dadurch gekennzeichnet, dass** sie das Exopolysaccharid-abbauende Enzym nach Anspruch 1 enthält.

3.  Zusammensetzung nach Anspruch 2, **dadurch gekennzeichnet, dass** die Zusammensetzung ferner mindestens ein Enzym aus der Gruppe bestehend aus Polysaccharidasen, Proteasen, Lipasen und Glykoproteasen enthält.

4.  Zusammensetzung nach Anspruch 3, **dadurch gekennzeichnet, dass** das Enzym eine Protease ist, die aus der Gruppe bestehend aus Serin-Endoprotease, die von *Bacillus licheniformis* produziert wird, Protease Typ XXI aus *Streptomyces griseus,* Metalloprotease aus *Bacillus polymyxa* Typ IX und As*pergillus*-Säure-Proteinase aus *Aspergillus saitoi* ausgewählt ist.

5.  Verfahren zur Herstellung des Enzyms nach Anspruch 1, **dadurch gekennzeichnet, dass** man den *Streptomyces-Stamm* ATCC 55601 in einem geeigneten Medium kultiviert.

6.  Verfahren zur Herstellung des Enzyms nach Anspruch 1, **dadurch gekennzeichnet, dass** man eine Mutante, eine Variante oder eine Transformante des Streptomyces-Stamms ATCC 55601, der das Enzym nach Anspruch 1 produziert, in einem geeigneten Medium kultiviert.

7.  Verwendung des Enzyms nach Anspruch 1 und/oder einer Zusammensetzung nach den Ansprüchen 2 bis 4 zur Reduktion, Entfernung und/oder Verhinderung eines Biofilms auf Oberflächen wässriger Systeme, **dadurch gekennzeichnet, dass** die Verwendung nicht die Behandlung des menschlichen oder tierischen Körpers durch Therapie, die am menschlichen oder tierischen Körper vorgenommen wird, betrifft.

8.  Verwendung nach Anspruch 7, **dadurch gekennzeichnet, dass** das wässrige System ein offenes oder geschlossenes industrielles Prozesswasser- oder Kühlwassersystem ist.

9.  Verwendung nach Anspruch 8, **dadurch gekennzeichnet, dass** das industrielle Prozesswassersystem ein offener oder geschlossener Wasserkreislauf in einer Papierfabrik ist.

10. Verwendung nach Anspruch 7, **dadurch gekennzeichnet, dass** die Oberflächen Ultrafiltrations- oder Dialyse-Membranen sind.

11. Verwendung des Enzyms nach Anspruch 1 und/oder einer Zusammensetzung nach den Ansprüchen 2 bis 4 zum Abbau von Exopolysacchariden, **dadurch gekennzeichnet, dass** die Verwendung nicht die Behandlung des menschlichen oder tierischen Körpers durch Therapie, die am menschlichen oder tierischen Körper vorgenommen wird, betrifft.

12. *Streptomyces*-Stamm ATCC 55601.

13. Mutante, Variante oder Transformante des *Streptomyces-Stamms* nach Anspruch 12, der ein Enzym nach Anspruch 1 produziert.

**Revendications**

1.  Enzyme capable de dégrader des exopolysaccharides, pouvant être obtenue par culture dans un milieu approprié, de la souche de *Streptomyces* ATCC 55601, **caractérisée en ce qu'**elle a un poids moléculaire d'environ 100 kDa par détermination en SDS-PAGE dans des conditions dénaturantes, et **en ce qu'**elle est capable de dégrader l'acide colanique.

2.  Composition enzymatique pour la réduction, l'élimination et/ou la prévention de biofilm sur les surfaces de systèmes aqueux, **caractérisée en ce qu'**elle comprend l'enzyme capable de dégrader des exopolysaccharides selon la revendication 1.

3.  Composition selon la revendication 2. **caractérisée en ce qu'**elle comprend aussi au moins une enzyme issue du groupe comprenant les polysaccharidases, les protéases, les lipases et les glycoprotéases.

4.  Composition selon la revendication 3, **caractérisée en ce que** l'enzyme est une protéase choisie au sein du groupe comprenant l'endoprotéase de la sérine produite par le *Bacillus lichenformis,* la protéase type XXI du *Streptomyces griseus,* la métalloprotéase de *Bacillus polymyxa* type IX et de la protéinase acide d'*Aspergillus* de l'*Aspergillus saitoi*.

**5.** Procédé de production de l'enzyme selon la revendication 1, **caractérisé en ce que** la souche de *Streptomyces* ATCC 55601 est cultivée dans un milieu approprié.

**6.** Procédé de production de l'enzyme selon la revendication 1, **caractérisé en ce qu'**un mutant, un variant ou un transformant de la souche de *Streptomyces* ATCC 55601 qui produit ladite enzyme selon la revendication 1, est cultivée dans un milieu approprié.

**7.** Utilisation de l'enzyme selon la revendication 1 et/ou de la composition selon les revendications 2 à 4 pour la réduction, l'élimination et/ou la prévention de biofilm sur les surfaces de systèmes aqueux, dans laquelle ladite utilisation n'est pas destinée au traitement du corps d'un humain ou d'un animal par une thérapie pratiquée sur ledit corps humain ou animal.

**8.** Utilisation selon la revendication 7, **caractérisée en ce que** le système aqueux est un système ouvert ou fermé d'eau pour usage industriel ou d'eau de refroidissement.

**9.** Utilisation selon la revendication 8, **caractérisée en ce que** le système d'eau pour usage industriel est un système cyclique d'eau ouvert ou fermé dans une usine de fabrication de papier.

**10.** Utilisation selon la revendication 7, **caractérisée en ce que** les surfaces sont des membranes d'ultrafiltration ou de dialyse.

**11.** Utilisation de l'enzyme selon la revendication I et/ou de la composition selon les revendications 2 à 4, pour la dégradation d'exopolysaccharides, dans laquelle ladite utilisation n'est pas destinée au traitement du corps d'un humain ou d'un animal par une thérapie pratiquée sur ledit corps humain ou animal.

**12.** Souche de *Streptomyces* ATCC 55601.

**13.** Mutant, variant ou transformant de la souche de *Streptomyces* selon la revendication 12, qui produit l'enzyme selon la revendication 1.

Fig.1

EP 0 820 516 B1

Fig. 2

Fig.3 :Removal results

Legend:
- ×---× Minimum value
- □— Biofilm mass
- ◇····· Maximum value

Biofilm mass ( mg/cm² )

Time ( h )

Fig.4 : Prevention results

EP 0 820 516 B1

Fig.5

EP 0 820 516 B1

Fig. 6

HYDROLYSIS OF COLANIC ACID BY EPS'ase
( 24 hrs, 35°C )

EP 0 820 516 B1